(19)

Europäisches
Patentamt
European
Patent Office
Office européen
des brevets

(11)  **EP 4 098 251 B1**

(12)  # EUROPEAN PATENT SPECIFICATION

(45) Date of publication and mention
of the grant of the patent:
**01.04.2026  Bulletin 2026/14**

(21) Application number: **21753821.4**

(22) Date of filing: **09.02.2021**

(51) International Patent Classification (IPC):
*A61K 9/00* *(2006.01)*        *A61K 9/127* *(2025.01)*
*A61K 31/136* *(2006.01)*       *A61P 35/00* *(2006.01)*
*A61P 35/04* *(2006.01)*

(52) Cooperative Patent Classification (CPC):
**A61P 35/00; A61K 9/0019; A61K 9/127;**
**A61K 31/136; A61P 35/04**

(86) International application number:
**PCT/CN2021/076210**

(87) International publication number:
**WO 2021/160115 (19.08.2021 Gazette 2021/33)**

(54) **MITOXANTRONE HYDROCHLORIDE LIPOSOME FOR USE IN TREATING BREAST CANCER**

MITOXANTRON-HYDROCHLORID-LIPOSOM ZUR VERWENDUNG IN DER BEHANDLUNG VON
BRUSTKREBS

LIPOSOME DE CHLORHYDRATE DE MITOXANTRONE POUR L'UTILISATION DANS LE
TRAITEMENT DU CANCER DU SEIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **10.02.2020  CN 202010083745**

(43) Date of publication of application:
**07.12.2022  Bulletin 2022/49**

(73) Proprietor: **CSPC Zhongqi Pharmaceutical
Technology
(Shijiazhuang) Co., Ltd.
Shijiazhuang, Hebei 050035 (CN)**

(72) Inventors:
  • **LI, Chunlei
    Shijiazhuang, Hebei 050035 (CN)**
  • **WANG, Xiaodong
    Shijiazhuang, Hebei 050035 (CN)**
  • **YANG, Xiugao
    Shijiazhuang, Hebei 050035 (CN)**
  • **WANG, Lingling
    Shijiazhuang, Hebei 050035 (CN)**
  • **DU, Yanling
    Shijiazhuang, Hebei 050035 (CN)**
  • **JIN, Qiushuang
    Shijiazhuang, Hebei 050035 (CN)**
  • **WANG, Shixia
    Shijiazhuang, Hebei 050035 (CN)**
  • **WANG, Xiaoli
    Shijiazhuang, Hebei 050035 (CN)**

(74) Representative: **Gille Hrabal
Partnerschaftsgesellschaft mbB
Patentanwälte
Brucknerstraße 20
40593 Düsseldorf (DE)**

(56) References cited:
**WO-A1-2018/048752      CN-A- 101 209 243
CN-A- 103 622 909        CN-A- 104 971 044
CN-A- 105 287 383        CN-A- 105 287 383
CN-A- 110 711 178**

• **LU B ET AL: "Solid lipid nanoparticles of
mitoxantrone for local injection against breast
cancer and its lymph node metastases",
EUROPEAN JOURNAL OF PHARMACEUTICAL
SCIENCES, ELSEVIER AMSTERDAM, NL, vol. 28,
no. 1-2, 1 May 2006 (2006-05-01), pages 86 - 95,
XP027996675, ISSN: 0928-0987, [retrieved on
20060501]**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

• **POUJOL SYLVAIN ET AL.: "Effent of Mitoxantrone Liposomes on Multidrug-Resistant Breast Cancer Cells,", ANTICANCER RESEARCH, vol. 19, 31 December 1999 (1999-12-31), pages 3327 - 3331, XP009529784, ISSN: 0250-7005**

**Description**

CROSS-REFERENCE TO RELATED PATENT APPLICATION

The present patent application claims priority to Chinese Patent Application No. 202010083745.4 filed with the China National Intellectual Property Administration on Feb. 10, 2020, entitled "USE OF MITOXANTRONE HYDROCHLORIDE LIPOSOME FOR TREATING BREAST CANCER'.

The present application also refers to Chinese Patent Application No. 200610102339.8 filed on Dec. 29, 2006, and PCT Application No. WO2008/080367A1 filed on Dec. 29, 2007.

TECHNICAL FIELD

The present disclosure relates to the field of anti-tumor therapies, and particularly to a mitoxantrone hydrochloride liposome for use in the treatment of breast cancer.

BACKGROUND

Breast cancer is one of the most common malignancies in women, with global statistics showing that about 1.7 million people (25% of all female cancer patients) are diagnosed with breast cancer and about 520 thousand people (15% of all female cancer patients) die from this disease each year. Breast cancer is a systemic disease, and in the early and middle stages of the disease, breast cancer cells can enter the circulatory system and metastasize throughout the body. At present, the treatment of breast cancer mainly includes surgery, chemoradiotherapy and endocrine therapy. However, advanced breast cancer shows a low survival rate and is generally refractory. The pathological types of breast cancer include luminal A, luminal B, normal breast-like, human epidermal growth factor receptor 2 (HER2) overexpression and basal-like 2 breast cancer. Triple-negative breast cancer is a type of breast cancer that is negative for estrogen receptor, progesterone receptor and HER2, and accounts for about 15%-20% of all newly diagnosed cases of breast cancer. HER2 overexpression and amplification have been found in various solid tumors, including breast cancer, gastric cancer and the like. Treatments with trastuzumab targeting HER2 in combination with other treatment regimens such as chemotherapy have demonstrated significant efficacy in HER2-positive patients. However, for the patients with advanced HER2-negative breast cancer who have failed endocrine therapy, chemotherapy is still the prominent treatment. The "Chinese Guidelines for Breast Cancer Diagnosis and Treatment" (2018 Edition) issued by the Chinese Health Commission, states that the commonly used chemotherapeutic agents for advanced breast cancer include anthracyclines (epirubicin, doxorubicin and doxorubicin), taxanes, vinorelbine, capecitabine, gemcitabine, platinum-based chemotherapeutics, etc. However, due to the severe toxicity and side effects, the use of chemotherapeutics in clinical practice is limited, and the treatment to patients may be discontinued due to intolerability to severe side effects. Therefore, it is particularly important to develop a chemotherapeutic regimen with better efficacy and lower toxicity.

Mitoxantrone hydrochloride is an anthraquinone chemotherapy drug, and the mitoxantrone hydrochloride injection has been approved by FDA for multiple sclerosis, prostate cancer and acute myeloid leukemia. Mitoxantrone hydrochloride has been approved in China for patients with lymphoma, leukemia, breast cancer and the like, and for adult patients, the recommended dose is 12-14 mg/m$^2$ once every 3-4 weeks, or 4-8 mg/m$^2$ once daily for 3-5 days with an interval of 2-3 weeks for monotherapies, or 5-10 mg/m$^2$ once for combination therapies. Mitoxantrone, as an anthracycline, induces adverse effects mainly including cardiotoxicity, myelosuppression, gastrointestinal reactions and the like. Myelosuppression and gastrointestinal reactions can be solved by adminstering suitable drugs, but the cardiotoxicity usually brings about serious consequences and is the most severe adverse effect of anthracyclines. Clinical studies and practical observations have shown that the cardiotoxicity caused by anthracyclines are mostly progressive and irreversible, especially anthracyclines can easily cause cardiac injury in the first use. Cardiotoxicity is a long-term cumulative dose-limiting toxicity and a common concern for clinicians. Therefore, despite the approval for breast cancer, mitoxantrone has not been recommended as a treatment for breast cancer by authoritative clinical medication guidelines.

Also, no controlled clinical trials of the mitoxantrone hydrochloride liposome disclosed herein and a common mitoxantrone hydrochloride formulation in the population with advanced breast cancer have been reported. Moreover, there is no published regimen of the mitoxantrone hydrochloride liposome for the treatment of patients with breast cancer and advanced breast cancer, especially patients with advanced HER-2-negative breast cancer.

Liposome is a novel drug carrier. Studies have shown its passive targeting property and it can alter the distribution of encapsulated drugs *in vivo* and allow the drugs to accumulate mainly in tissues and organs such as liver, spleen, lung, bone marrow and the like, thereby improving the therapeutic index of the drugs, reducing the therapeutic dose of the drugs and lowering the toxicity of the drugs. These properties make the use of liposome-loaded drugs highly valued in the research of anti-tumor drugs. Researchers have conducted studies on liposomal formulations of mitoxantrone. For example, a mitoxantrone liposome is disclosed in Chinese Patent Application No. 200610102339.8 filed on Dec. 29, 2006, and PCT Application WO2008/080367A1 filed on Dec. 29, 2007, as well as in CN 105 287 383 A. Studies have shown that compared with free form of mitoxantrone, the *in vivo* pharmacokinetics and tissue distribution of the drug have changed due to the encapsulation of liposomes, which leads to, for example, prolonged effective period in blood, reduced amount in normal tissues, increased accumulation in tumor tissues, lower toxicity, and better anti-tumor efficacy at a lower dose. Mitoxantrone liposome seems to be a potential therapeutic approach for breast cancer, especially advanced breast cancer.

As the originator of the innovative drug-mitoxantrone liposome, CSPC Pharmaceutical Group has previously explored and investigated the mitoxantrone liposome for treating solid tumors and lymphomas. However, the mitoxantrone liposome is a special dosage form different from the common injections due to its complicated absorption, distribution and metabolism in the body, making it difficult to be bridged from one indication to another. It is well known in the art that different tumors vary in drug sensitivity. Previous studies have shown that the same drug may be administered in different regimens for different indications. For example, Doxil (doxorubicin hydrochloride liposome) has been approved by the FDA for three indications: (1) ovarian cancer, with a recommended dose of 50 mg/m$^2$, administered intravenously once every 4 weeks; (2) Kaposi's sarcoma, with a recommended dose of 20 mg/m$^2$, administered intravenously every 3 weeks; and (3) multiple myeloma, with a recommended dose of 30 mg/m$^2$, administered intravenously on the fourth day after administration of bortezomib. For another example, Abraxane (albumin-bound paclitaxel for injection) has also been approved by the FDA for three indications: (1) metastatic breast cancer, with a recommended dose of 260 mg/m$^2$, administered by intravenous drip in 30 min once every 3 weeks; (2) non-small cell lung cancer, with a recommended dose of 100 mg/m$^2$, administered by intravenous drip in 30 min on days 1, 8 and 15 in every 21-day treatment cycle, where carboplatin is administered immediately after the administration of albumin-bound paclitaxel for injection on day 1, i.e., once every 21 days; and (3) pancreatic cancer, with a recommended dose of 125 mg/m$^2$, administered by intravenous drip in 30-40 minutes on days 1, 8 and 15 in every 28-day treatment cycle, where gemcitabine is administered immediately after each dose of albumin-bound paclitaxel for injection. For still another example, the starting dose of AmBisome (amphotericin B liposomes for injection) for the treatment of the following indications is: (1) empiric therapy: a recommended dose of 3 mg/kg/day; (2) systemic fungal infection (e.g., Aspergillus, Candida and Cryptococcus): a recommended dose of 3-5 mg/kg/day; (3) cryptococcal meningitis in HIV-infected patients: a recommended dose of 6 mg/kg/day (administered on days 1-5) and 3 mg/kg/day (administered on days 4 and 21); and (4) visceral leishmaniasis in immunocompromised patients: 4 mg/kg/day (administered on days 1-5) or 4 mg/kg/day (administered on days 10, 17, 24, 31 and 38). The dose and administration parameters can be individualized according to the actual condition of patients to reach maximized efficacy and the minimized toxicity or adverse effect. It can be seen that a drug may have different safe and effective doses for different indications. The dose and administration parameters can be individualized according to the disease and the actual condition of patients to reach maximized efficacy and the minimized toxicity or adverse effect for safe and effective disease treatment.

In summary, despite the approval for treating breast cancer, mitoxantrone has not been recommended as a treatment for breast cancer by authoritative clinical medication guidelines, and there is no previous experience in the treatment of breast cancer with mitoxantrone, making it difficult to find reliable reference for the safety and efficacy of mitoxantrone liposome monotherapy for treating breast cancer. The mitoxantrone liposome is a special dosage form different from the common injections due to its complicated absorption, distribution and metabolism in the body, making it difficult to expand from one indication to another, especially among different cancers. Moreover, mitoxantrone as a drug is mainly metabolized in the liver, and the main adverse effects include cardiotoxicity, myelosuppression, impaired liver function and the like. Liposome-loaded drugs may further accumulate in tissues such as liver, spleen, bone marrow and the like, which will lead to a significant increase in the burden of liver, spleen and bone marrow, and whether this will affect the efficacy and safety of the treatment is difficult to predict. Therefore, it is necessary to conduct systematic studies on whether the mitoxantrone liposome is suitable for the treatment of breast cancer, especially advanced breast cancer, to determine a safe and effective dose of the mitoxantrone liposome and to provide reference for clinical practice.

SUMMARY

The present disclosure provides a mitoxantrone hydrochloride liposome for use in the treatment of breast cancer.

According to the invention, the breast cancer is advanced recurrent or metastatic breast cancer that is unsuitable for or resistant to endocrine therapy, HER2 negative or refractory to HER2 targeted therapy.

Preferably, the mitoxantrone hydrochloride liposome plays a role of a sole active ingredient.

Preferably, the liposome is in a dosage form for injection, including a liquid for injection, a powder for injection, a tablet for injection.

Preferably, the dosage form is a liquid for injection.

When the dosage form is a liquid for injection, the dosage form comprises 0.5-5 mg/mL, preferably 1-2 mg/mL, and more preferably 1 mg/mL of the active ingredient, on the basis of mitoxantrone.

The present disclosure further provides, but does not claim, a method for treating breast cancer, comprising:

administering to a patient with breast cancer a therapeutically effective amount of a mitoxantrone hydrochloride liposome. Preferably, the therapeutically effective amount refers to 8-30 mg/m$^2$, more preferably 12-20 mg/m$^2$ or 16-30 mg/m$^2$, on the basis of mitoxantrone, for a specific example, 12 mg/m$^2$, 14 mg/m$^2$, 16 mg/m$^2$, 18 mg/m$^2$ or 20 mg/m$^2$.

Preferably, the route of administration described herein is intravenous administration. Preferably, the frequency of administration is once every 4 weeks. Preferably, for each intravenous administration, the dripping administration time of the liposomal pharmaceutical formulation is 30-120 min, preferably 55-120 min or 60-120 min, further preferably not less than 60 min, and more preferably 60 ± 5 min. The liposomal pharmaceutical formulation is in a dosage form for injection of the mitoxantrone hydrochloride liposome, including a liquid for injection, a powder for injection, a tablet for injection, and the like. Preferably, the patient receives a total dose of mitoxantrone of no more than 200 mg/m$^2$, preferably no more than 160 mg/m$^2$, further preferably no more than 140 mg/m$^2$, and more preferably no more than 120 mg/m$^2$.

The present disclosure provides use of the liposomal pharmaceutical formulation described above for manufacturing a medicament for the treatment of breast cancer, wherein each patient receives a total dose of the liposomal pharmaceutical formulation of no more than 200 mg/m$^2$, preferably no more than 160 mg/m$^2$, further preferably no more than 140 mg/m$^2$, and more preferably no more than 120 mg/m$^2$, on the basis of mitoxantrone. The liposomal pharmaceutical formulation is in a dosage form for injection of the mitoxantrone hydrochloride liposome, including a liquid for injection, a powder for injection, a tablet for injection, and the like.

The present invention provides a mitoxantrone hydrochloride liposome for use in treating breast cancer in a patient.

In some examples, the liposome is in a dosage form for injection, including a liquid for injection, a powder for injection, a tablet for injection. When the dosage form is a liquid for injection, the dosage form comprises 0.5-5 mg/mL, preferably 1-2 mg/mL, and more preferably 1 mg/mL of the active ingredient, on the basis of mitoxantrone.

In some examples, the liposome is used alone for treating breast cancer in the patient.

In some examples, the therapeutically effective amount of the liposomes is 8-30 mg/m$^2$, more preferably 12-20 mg/m$^2$ or 16-30 mg/m$^2$, on the basis of mitoxantrone, for a specific example, 12 mg/m$^2$, 14 mg/m$^2$, 16 mg/m$^2$, 18 mg/m$^2$ or 20 mg/m$^2$.

In some examples, the route of administration of the liposome is intravenous administration. Preferably, the frequency of administration is once every 4 weeks. Preferably, for each intravenous administration, the dripping administration time of the liposome is 30-120 min, preferably 55-120 min or 60-120 min, further preferably not less than 60 min, and more preferably 60 ± 5 min.

In some examples, the patient receives a total dose of mitoxantrone of no more than 200 mg/m$^2$, preferably no more than 160 mg/m$^2$, further preferably no more than 140 mg/m$^2$, and more preferably no more than 120 mg/m$^2$.

In the context of the present disclosure, the "total administered dose of mitoxantrone" refers to the sum of the amounts of all mitoxantrone medicaments received by the patient, including mitoxantrone hydrochloride liposome disclosed herein, mitoxantrone hydrochloride injection, and other mitoxantrone formulations, on the basis of mitoxantrone.

In the context of the present disclosure, unless otherwise specified, the dose is on the basis of mitoxantrone.

In the context of the present disclosure, the mitoxantrone hydrochloride liposome can be manufactured by conventional methods in the art, and can be those manufactured by any of the methods disclosed in the prior art, such as the method disclosed in Patent No. WO2008/080367A1

According to the invention, the mitoxantrone hydrochloride liposome has a particle size of about 60 nm and comprises: 1) mitoxantrone as active ingredient, which can form a hardly soluble precipitate with multivalent counterions in the liposome, and 2) a phospholipid bilayer containing hydrogenated soybean lecithin, cholesterol and polyethylene glycol 2000-modified distearoyl phosphatidyl ethanolamine in a mass ratio of 3:1:1, and the counterion is sulfate ion. Alternatively, the phospholipid bilayer of the liposome contains hydrogenated soybean lecithin, cholesterol and polyethylene glycol 2000-modified distearoyl phosphatidyl ethanolamine in a mass ratio of 3:1:1, the particle size is about 40-60 nm, the counterion is sulfate ion, and the mass ratio of HSPC:Chol:DSPE-PEG2000:mitoxantrone is 9.58:3.19:3.19:1.

In the context of the present disclosure, the manufacturing process of the mitoxantrone liposome is as follows: HSPC (hydrogenated soybean phosphatidylcholine), Chol (cholesterol) and DSPE-PEG2000 (polyethylene glycol 2000-modified distearoyl phosphatidyl ethanolamine) are weighed in a mass ratio of 3:1:1 and dissolved in 95% ethanol to obtain a clear solution. The solution of phospholipids in ethanol is mixed with a 300 mM ammonium sulfate solution, and the resulting mixture is shaken for hydration at 60-65 °C for 1 h to obtain a heterogeneous multivesicular liposome. The particle size of the liposome is then reduced using a microfluidization homogenizer. The obtained sample is 200-fold diluted with a 0.9% NaCl solution and then detected with NanoZS. The average particle size of the particles is about 60 nm, with the main peaks are concentrated between 40-60 nm. The ammonium sulfate in the external phase of the blank liposome is then removed using an ultrafiltration device, and the external phase is replaced with 290 mM sucrose and 10 mM glycine to form a transmembrane ammonium sulfate gradient. A mitoxantrone hydrochloride solution (10 mg/mL) is added to the blank liposome at a lipid/drug ratio of 16:1, and the drug is loaded at 60-65 °C. After 1 h of incubation, it could be determined by the gel size exclusion chromatography that the encapsulation efficiency is about 100%. The product obtained from this formulation is named PLM 60. PLLM60 contains HSPC, Chol, DSPE-PEG2000 and mitoxantrone in a weight ratio of 9.58:3.19:3.19:1, and the osmotic pressure of the sucrose-glycine solution is close to the physiological value.

In the context of the present disclosure, the patient with advanced recurrent breast cancer refers to a patient who has been treated with prior anti-breast cancer therapy but experienced recurrence. The patient has received prior treatment with medications including, but not limited to: 5-FU + pharmorubicin + cyclophosphamide, gemcitabine + cisplatin, paclitaxel, eribulin, paclitaxel + epirubicin, gemcitabine + paclitaxel, capecitabine, toremifene citrate, Herceptin (trastuzumab) + paclitaxel + carboplatin, Herceptin (trastuzumab), vinorelbine + Herceptin (trastuzumab), paclitaxel + carboplatin, epirubicin + cyclophosphamide, exemestane, letrozole + metformin, vinorelbine, docetaxel + capecitabine, letrozole, epirubicin + cyclophosphamide + docetaxel, exemestane + zoledronic acid, paclitaxel polymer micelle for injection, gemcitabine, gemcitabine + carboplatin, cyclophosphamide + docetaxel, cyclophosphamide + epirubicin + fluorouracil, tamoxifen, radiotherapy, docetaxel + carboplatin, vinorelbine + capecitabine, fulvestrant, paclitaxel + gemcitabine, capecitabine + Pseudomonas Aeruginosa Preparation, etoposide + disodium clodronate, cyclophosphamide, oxaliplatin + calcium folinate + fluorouracil, cyclophosphamide + epirubicin + fluorouracil + docetaxel, docetaxel + epirubicin, vinorelbine + cisplatin, docetaxel, famitinib, anastrozole, oxaliplatin + calcium folinate + fluorouracil + fluorouracil, apatinib, cyclophosphamide + tegafur + doxorubicin liposome, goserelin, goserelin + anastrozole, leuprorelin + exemestane, fluorouracil + epirubicin + cyclophosphamide + docetaxel, etoposide, docetaxel + capecitabine, toremifene, anastrozole + everolimus, paclitaxel + pharmorubicin, oxaliplatin + calcium folinate + fluorouracil, fluorouracil, tegafur + gimericil + oteracil potassium, cyclophosphamide + pharmorubicin + 5-fluorouracil, metformin + letrozole, Gainuo (Vinorelbine tartrate injection), etoposide, cyclophosphamide + pirarubicin + fluorouracil, proton pump inhibitor + docetaxel, anastrozole, tamoxifen citrate, lapatinib tablet + capecitabine tablet, letrozole + goserelin, metformin + exemestane, vinorelbine + capecitabine + Avastin, goserelin, anastrozole + goserelin, exemestane + goserelin, everolimus + tamoxifen, albumin-bound paclitaxel + gemcitabine + recombinant human endostatin + docetaxel + gemcitabine + recombinant human endostatin, vinflunine + capecitabine, sunitinib maleate, irinotecan liposome, epirubicin, doxorubicin, pharmorubicin and pirarubicin.

DETAILED DESCRIPTION

Example 1. Mitoxantrone hydrochloride liposome monotherapy for treatment of breast cancer

I. Trial:

The study is a randomized, open-labeled, active controlled, single center study to evaluate the safety and efficacy of the mitoxantrone hydrochloride liposome injection in patients with advanced recurrent or metastatic breast cancer.

1. Administration

Table 1. Specific Administration

| Drug | Dosage and route | Time and treatment cycle |
|---|---|---|
| Mitoxantrone hydrochloride liposome injec- tion | 20 mg/m$^2$, i.v. d1 | Continuously i.v. drip for 1 h, once every 4 weeks |
| Mitoxantrone hydrochloride injection | 14mg/m$^2$, i.v. d1 | Continuously i.v. drip for 30 min, once every 4 weeks |

Investigational product: mitoxantrone hydrochloride liposome injection (PLM60 (10 mg / 10 mL / vial)) was added to 250 mL of 5% glucose at a dose of 20 mg/m$^2$ and was administered intravenously over 1 h.
Reference product: mitoxantrone hydrochloride injection (5 mg / 5 mL / vial, manufactured by Sichuan Sunnyhope Pharmaceutical, Inc., supplied by CSPC ZHONGQI Pharmaceutical Technology (Shijiazhuang) Co., Ltd., Lot No. 1501201) was added to 100 mL of 5% glucose injection at a dose of 14 mg/m$^2$ and was administered intravenously over 30 min.

2. Inclusion criteria:

The inclusion criteria for the patient in this study are as follows:

1) Voluntary participation and written informed consent;

2) Aged 18 to 75 years (inclusive), female;

3) Pathologically and/or cytologically confirmed recurrent, metastatic breast cancer; and failed for at least two lines of chemotherapies to the recurrent or metastatic lesions;

4) Suitable for chemotherapy;

5) HER2 negative patients (definition: IHC 0 or 1+; IHC 2+ and FISH negative); HER2 positive patients unable to undergo anti-HER2 targeted therapy (definition: IHC 3+; or FISH positive);

6) Unsuitable for endocrine therapy or confirmed endocrine resistance;

7) Having at least one measurable lesion (according to RECIST 1.1 criteria, diameter $\geq$ 10 mm by spiral CT scan);

8) If there is an anthracycline-containing adjuvant chemotherapy, an interval of more than 12-month was required from the last dose of the prior adjuvant chemotherapy to the recurrence;

9) ECOG score of 0-2 (for ECOG scale, see Appendix 1), and an expected life expectancy of at least 3 months;

10) Normal cardiac function (NYHA class **I,** LVEF $\geq$ 50%);

11) All subjects of child-bearing age (fertile) must take medically well-known effective contraceptive measures;

12) The blood, liver and kidney function test results are in the following ranges:

$$\text{White Blood Cell (WBC)} \geq 3.0 \times 10^9/\text{L},$$

$$\text{Absolute Neutrophil Count (ANC)} \geq 1.5 \times 10^9/\text{L},$$

$$\text{PLT} \geq 75 \times 10^9/\text{L},$$

$$\text{Blood Hb} \geq 90 \text{ g/L},$$

$$\text{Tbil} \leq 1.5 \times \text{Upper Limit of Normal (ULN)},$$

$$\text{ALT and AST} \leq 2.5 \times \text{ULN}$$

(for subject with liver metastasis, $\leq$ 5 $\times$ ULN),

$$\text{Cr} \leq 1.5 \times \text{ULN}.$$

Note: Anti-HER 2 targeted therapy may not be available for HER2-positive patients due to unaffordability, toxicity, or tolerance reasons. HER2-positive patients who were unable to undergo anti-HER 2 targeted therapy could be enrolled.

3. Safety assessment:

The safety assessment indicators included: vital signs, adverse events, laboratory tests (complete blood test, routine urinalysis, blood biochemistry, electrocardiogram, echocardiogram), and early withdrawal. Safety assessment criteria: NCI-CTC 4.0.

4. Efficacy evaluation:

A tumor assessment was performed at baseline.

During the treatment period, the efficacy evaluation was performed once every two cycles; in addition, when the efficacy evaluation result was PR or CR, the efficacy should be radiologically confirmed after 4 weeks. After the completion of the treatment, the evaluation was performed every 3 months until disease progression. Tumor evaluation was performed according to RECIST 1.1 criteria.

Primary efficacy endpoint: objective response rate (ORR), referring to the ratio of subjects reaching PR and CR.

Secondary efficacy endpoint: progression-free survival (PFS), referring to the time from the start of randomization to disease progression or death.

II. Trial results

1. Efficacy analysis:

In this study, 60 eligible breast cancer patients were randomized to the treatment and control groups with 30 subjects in each. Of the 60 subjects, 42 subjects were treated for < 4 cycles, of whom 19 were in the treatment group and 23 were in the control group; 18 subjects received more than 4 cycles of treatment, of whom 11 were in the treatment group and 7 were in the control group.

Treatment group: among the 30 breast cancer subjects, 4 had partial response (PR), and 11 had stable disease (SD), showing an ORR of 13.3% (4/30), and a disease control rate (PR + SD) of 50% (15/30);

Control group: among the 30 breast cancer subjects, 2 had partial response (PR), and 7 had stable disease (SD), showing an ORR of 6.7% (2/30), and a disease control rate (PR + SD) of 30% (9/30). In FAS, the median PFS was 2.3 months for the treatment group and 1.86 months for the control group, and the PFS in the treatment group was numerically higher than the control group. In FAS, the 3-, 6- and 12-month progression-free survival rates of the two groups (treatment group *vs.* control group) were 43.3% *vs.* 26.7%, 21.9% *vs.* 20%, and 16.4% *vs.* 0%, respectively.

The objective response rates (13.3% vs. 6.7%) and disease control rates (50% vs. 30%) of the treatment group were numerically superior to those of the control group. The efficacy of the mitoxantrone hydrochloride liposome monotherapy for treating breast cancer is detailed in the following tables.

Table 2. Inter-group comparative analysis of best overall response of the two groups

| Efficacy evaluation | FAS (N=60) | | | | PPS (N=58) | | | |
|---|---|---|---|---|---|---|---|---|
| | Treatment group (n = 30) | Control group (n = 30) | Statistics | P value | Treatment group (n = 30) | Control group (n = 28) | Statistics | P value |
| Best overall response evaluation | | | 3.626 | 0.2818 | | | 1.932 | 0.4276* |
| PR | 4 (13.3%) | 2 (6.7%) | | | 4 (13.3%) | 2 (7.1%) | | |
| SD | 11 (36.7%) | 7 (23.3%) | | | 11 (36.7%) | 7 (28.6%) | | |
| PD | 15 (50.0%) | 19 (63.3%) | | | 15 (50.0%) | 19 (67.9%) | | |
| NA | 0(0.0) | 2(6.7%) | | | 0(0.0) | 0(0.0) | | |
| Note: Fisher's exact probability was used. | | | | | | | | |

Table 3. Median PFS and inter-group comparative analysis of the two groups

| Group | FAS (N=60) | | | PPS (N= 58) | | | | | | | Log-rank P value |
|---|---|---|---|---|---|---|---|---|---|---|---|
| Treatment group | 30 | 24(80.0) | 2.30 | 1.741, 3.910 | 0.565 | 30 | 24(80.0) | 2.30 | 1.741, 3.910 | | 0.689 |
| Control group | 30 | 25(83.3) | 1.86 | 1.741, 2.398 | | 28 | 23(82.14) | 1.86 | 1.741, 2.004 | | |

Table 4. Progression-free survival rate and 95% confidence intervals of the two groups

| PFS (Months) | FAS(N= 60) | | | | PPS(N=58 ) | | | |
| --- | --- | --- | --- | --- | --- | --- | --- | --- |
| | Treatment group | | Control group | | Treatment group | | Control group | |
| | Progression-free survival rate | 95%CI | Progression-free survival rate | 95%CI | Progression-free survival rate | 95%CI | Progression-free survival rate | 95%CI |
| 3 | 43.3 | 25.6-59.9 | 26.7 | 12.6-43.0 | 43.3 | 25.6-59.9 | 28.6 | 13.5-45.6 |
| 6 | 21.9 | 8.9-38.4 | 20.0 | 6.9-38.0 | 21.9 | 8.9-38.4 | 21.4 | 7.0-40.3 |
| 12 | 16.4 | 5.1-33.4 | 0 | 0 | 16.4 | 5.1-33.4 | 0 | 0 |

Note: The abbreviations above have the following meanings:

CR: complete response, defined explicitly according to the "Response Evaluation Criteria in Solid Tumors" (RECIST 1.1).

PR: partial response, defined explicitly according to the "Response Evaluation Criteria in Solid Tumors" (RECIST 1.1).

PD: progressive disease, defined explicitly according to the "Response Evaluation Criteria in Solid Tumors" (RECIST 1.1).

SD: stable disease, defined explicitly according to the "Response Evaluation Criteria in Solid Tumors" (RECIST 1.1).

FAS: full analysis set, statistical term.

PPS: per protocol set, statistical term.

$$\text{Overall response rate (ORR)} = (CR + PR)/\text{total number of evaluable patients} \times 100\%.$$

2. Safety analysis:

For the drug mitoxantrone, the most serious adverse event is cardiotoxicity. Clinical studies and practical observations have shown that the cardiotoxicity caused by anthracyclines are mainly progressive and irreversible. Especially anthracyclines can easily cause cardiac damage at first use. Cardiotoxicity isa common concern for clinicians since it is a long-term cumulative dose-limiting toxicity. The incidence of increased troponin T and the incidence of increased brain natriuretic peptide were used as indices for examining cardiotoxicity in this trial. The change of the cardiac troponin T level can reflect myocardium damage with high sensitivity; brain natriuretic peptide is a polypeptide hormone secreted by cardiomyocytes in the heart ventricles in response to stretching caused by increased ventricular blood volume and increased ventricular pressure.

From Table 5 it can be seen that: 1) The incidence of increased troponin T was 3.3% for the treatment group and 36.7% for the control group, with an extremely significant difference. Therefore, the liposome dosage form can significantly reduce the cardiotoxicity of mitoxantrone hydrochloride;

2) The incidence of increased brain natriuretic peptides was the same for the two groups (33.3% vs 33.3%). However, as can be seen from Tables 6 and 7, the levels of increase were different, and the number of cases and severity of the treatment group were significantly lower than those of the control group.

The overall incidence rate of adverse events was 100% for the treatment group and the control group, and the most common adverse events were hematological toxicity. Among the adverse events that occurred at least 30% in the groups, the incidences of decreased white blood cell count (86.7% vs 96.7%), decreased neutrophil count (80.0% vs 96.7%), increased conjugated bilirubin (53.3% vs 56.7%), increased aspartate aminotransferase (40.0% vs 53.3%) and increased troponin T (3.3% vs 36.7%) of the treatment group were lower than those in the control group, and the incidences of anemia (76.7% vs 46.7%), skin hyperpigmentation (66.7% vs 3.3%) and decreased platelet count (56.7% vs 53.3%) of the treatment group were higher than those in the control group. The trail results demonstrated that the mitoxantrone hydrochloride liposome has a trend superior to mitoxantrone hydrochloride in numerical values of objective response rate, disease control rate, progression-free survival and other indicators in treating advanced recurrent or metastatic breast cancer, and the cardiotoxicity of the mitoxantrone hydrochloride liposome is significantly lower than that of mitoxantrone hydrochloride.

Table 5. Incidence of adverse events

| Item | Treatment group (%) | Control group (%) |
|---|---|---|
| Increased troponin T | 1(3.3%) | 11(36.7%) |
| Increased brain natriuretic peptide | 10(33.3%) | 10(33.30%) |
| Decreased white blood cell count | 26(86.7%) | 29(96.70%) |
| Decreased neutrophil count | 24(80.0%) | 29(96.70%) |
| Anemia | 23(76.7%) | 14(46.70%) |
| Increased conjugated bilirubin | 16(53.3%) | 17(56.70%) |
| Decreased platelet count | 17(56.7%) | 16(53.30%) |
| Increased aspartate aminotransferase | 12(40.0%) | 16(53.30%) |
| Increased blood bilirubin | 9(30.0%) | 6(20.00%) |
| Fever | 7(23.3%) | 3(10.0%) |
| Increased alanine aminotransferase | 6(20.0%) | 8(26.70%) |

Table 6. Analysis of number of subjects with increased brain natriuretic peptide and severity

| | Treatment group (n = 30) | | | | Control group (n = 30) | | | |
|---|---|---|---|---|---|---|---|---|
| | Grade 1 | Grade 2 | Grade 3 | Grade 4 | Grade 1 | Grade 2 | Grade 3 | Grade 4 |
| Increased brain natriuretic peptide | 10(33.3%) | - | - | - | 9(30.0%) | 1(3.3%) | - | - |

Table 7. Analysis of number of cases with increased brain natriuretic peptide and severity

| | Treatment group (n = 30) | | | | Control group (n = 30) | | | |
|---|---|---|---|---|---|---|---|---|
| | Grade 1 | Grade 2 | Grade 3 | Grade 4 | Grade 1 | Grade 2 | Grade 3 | Grade 4 |
| Increased brain natriuretic peptide | 13 | - | - | - | 15 | 1 | - | - |

Appendix 1: ECOG scale

| Zubrod-ECOG-WHO (ZPS, 5 grades) | |
|---|---|
| Physical condition | Grade |
| Fully active | 0 |
| Symptomatic but completely ambulatory; able to carry out work of a light or sedentary nature | 1 |
| Able to tolerate tumor symptoms, capable of all self-care, <50% in bed during the day | 2 |
| Seriously symptomatic; >50% in bed during the day, but not bedbound, capable of only limited self-care | 3 |
| Bedbound | 4 |
| Death | 5 |

**Claims**

1. A mitoxantrone hydrochloride liposome for use in the treatment of breast cancer, wherein the breast cancer is advanced recurrent or metastatic breast cancer that is unsuitable for or tolerant to endocrine therapy, HER2 negative or refractory to HER2 targeted therapy;
wherein the mitoxantrone hydrochloride liposome has a particle size of about 60 nm and comprises: 1) mitoxantrone as active ingredient, which can form a hardly soluble precipitate with multivalent counterions in the liposome, and the counterions are sulfate ions; 2) a phospholipid bilayer containing hydrogenated soybean lecithin, cholesterol and polyethylene glycol 2000-modified distearoyl phosphatidyl ethanolamine in a mass ratio of 3:1:1; or , has a particle size of about 40-60 nm and comprises: 1) mitoxantrone as active ingredient, which can form a hardly soluble precipitate with multivalent counterions in the liposome, and the counterions are sulfate ions; 2) a phospholipid bilayer containing hydrogenated soybean lecithin, cholesterol and polyethylene glycol 2000-modified distearoyl phosphatidyl ethanolamine in a mass ratio of 3:1:1, and the mass ratio of HSPC:Chol:DSPE-PEG2000:mitoxantrone is 9.58:3.19:3.19:1, whereby the particle size is detected by NanoZS.

2. The mitoxantrone hydrochloride liposome for use in the treatment of breast cancer according to claim 1, wherein the mitoxantrone hydrochloride liposome plays a role of a sole active ingredient.

3. The mitoxantrone hydrochloride liposome for use in the treatment of breast cancer according to claim 1 or 2, wherein the liposome is in a dosage form for injection, including a liquid for injection, a powder for injection, a tablet for injection; preferably, the dosage form is a liquid for injection.

4. The mitoxantrone hydrochloride liposome for use in the treatment of breast cancer according to claim 3, wherein the dosage form is a liquid for injection and comprises 0.5-5 mg/mL, preferably 1-2 mg/mL, and more preferably 1 mg/mL of the active ingredient, on the basis of mitoxantrone.

**Patentansprüche**

1. Mitoxantron-Hydrochlorid-Liposom zur Verwendung bei der Behandlung von Brustkrebs, wobei der Brustkrebs fortgeschrittener, rezidivierender oder metastasierter Brustkrebs ist, der für endokrine Therapie ungeeignet oder dieser gegenüber tolerant, HER2-negativ oder gegenüber einer HER2-gerichteten Therapie refraktär ist;

wobei das eine Partikelgröße von etwa 60 nm aufweist und Folgendes umfasst: 1) Mitoxantron als Wirkstoff, das in der Lage ist, in dem Liposom einen schwerlöslichen Niederschlag mit mehrwertigen Gegenionen zu bilden, wobei die Gegenionen Sulfationen sind; 2) eine Phospholipid-Doppelschicht, die hydriertes Sojalecithin, Cholesterin und Polyethylenglykol-2000-modifiziertes Distearoylphosphatidylethanolamin in einem Massenverhältnis von 3:1:1 enthält; oder eine Partikelgröße von etwa 40-60 nm aufweist und Folgendes umfasst: 1) Mitoxantron als Wirkstoff, das in der Lage ist, in dem Liposom einen schwerlöslichen Niederschlag mit mehrwertigen Gegenionen zu bilden, wobei die Gegenionen Sulfationen sind; 2) eine Phospholipid-Doppelschicht, die hydriertes Sojalecithin, Cholesterin und Polyethylenglykol-2000-modifiziertes Distearoylphosphatidylethanolamin in einem Massenverhältnis von 3:1:1 enthält; und das Massenverhältnis von HSPC:Chol:DSPE-PEG2000:Mitoxantron 9,58:3,19:3,19:1 ist, wobei die Partikelgröße mittels NanoZS erfasst wird.

2. Mitoxantron-Hydrochlorid-Liposom zur Verwendung bei der Behandlung von Brustkrebs nach Anspruch 1, wobei das Mitoxantron-Hydrochlorid-Liposom eine Rolle eines einzigen Wirkstoffs spielt.

3. Mitoxantron-Hydrochlorid-Liposom zur Verwendung bei der Behandlung von Brustkrebs nach Anspruch 1 oder 2, wobei das Liposom in einer Darreichungsform zur Injektion ist, einschließlich einer Flüssigkeit zur Injektion, eines Pulvers zur Injektion oder einer Tablette zur Injektion; wobei die Darreichungsform vorzugsweise eine Flüssigkeit zur Injektion ist.

4. Mitoxantron-Hydrochlorid-Liposom zur Verwendung bei der Behandlung von Brustkrebs nach Anspruch 3, wobei die Darreichungsform eine Flüssigkeit zur Injektion ist und 0,5-5 mg/mL, bevorzugt 1-2 mg/mL und bevorzugter 1 mg/mL des Wirkstoffs auf der Basis von Mitoxantron umfasst.

## Revendications

1. Liposome de chlorhydrate de mitoxantrone pour l'utilisation dans le traitement du cancer du sein, dans lequel le cancer du sein est un cancer du sein avancé, récurrent ou métastatique qui ne convient pas à ou ne tolère pas une endocrinothérapie, HER2 négatif ou réfractaire à une thérapie ciblée HER2 ;
dans lequel le liposome de chlorhydrate de mitoxantrone présente une taille de particules d'environ 60 nm et comprend : 1) de la mitoxantrone comme ingrédient actif, qui peut former un précipité peu soluble avec des contre-ions multivalents dans le liposome, et les contre-ions sont des ions sulfate ; 2) une bicouche phospholipidique contenant de la lécithine de soja hydrogénée, du cholestérol et de la distéaroyl phosphatidyléthanolamine modifiée par du polyéthylène glycol 2000 selon un rapport massique de 3:1:1 ; ou, présente une taille de particules d'environ 40 à 60 nm et comprend : 1) de la mitoxantrone comme ingrédient actif, qui peut former un précipité peu soluble avec des contre-ions multivalents dans le liposome, et les contre-ions sont des ions sulfate ; 2) une bicouche phospholipidique contenant de la lécithine de soja hydrogénée, du cholestérol et de la distéaroyl phosphatidyléthanolamine modifiée par du polyéthylène glycol 2000 selon un rapport massique de 3:1:1, et le rapport massique de HSPC:Chol:DSPE-PEG2000:mitoxantrone est de 9,58:3,19:3,19:1, moyennant quoi la taille de particules est détectée par NanoZS.

2. Liposome de chlorhydrate de mitoxantrone pour l'utilisation dans le traitement du cancer du sein selon la revendication 1, dans lequel le liposome de chlorhydrate de mitoxantrone joue le rôle d'un seul ingrédient actif.

3. Liposome de chlorhydrate de mitoxantrone pour l'utilisation dans le traitement du cancer du sein selon la revendication 1 ou 2, dans lequel le liposome se présente sous une forme posologique injectable, notamment un liquide injectable, une poudre injectable, un comprimé injectable ; de préférence, la forme posologique est un liquide injectable.

4. Liposome de chlorhydrate de mitoxantrone pour l'utilisation dans le traitement du cancer du sein selon la revendication 3, dans lequel la forme posologique est un liquide injectable et comprend 0,5 à 5 mg/mL, de préférence 1 à 2 mg/mL, et de manière davantage préférée 1 mg/mL de l'ingrédient actif, sur la base du mitoxantrone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010083745
- CN 200610102339
- WO 2008080367 A1
- CN 105287383 A

**Non-patent literature cited in the description**

- Chinese Guidelines for Breast Cancer Diagnosis and Treatment. 2018